# FASCICULE DE BREVET EUROPEEN

(11) **EP 0 534 844 B1**
(45) Date de publication et mention de la délivrance du brevet: **07.02.1996**
(21) Numéro de dépôt: 92402607.3
(22) Date de dépôt: 23.09.1992
(51) Int. Cl.: C07D 215/42, A61K 31/47

(54) **Nouveaux dérivés de la décahydroquinoléine, leur procédé de préparation, les intermédiaires de préparation, leur application à titre de médicaments et les compositions les renfermant**
Neue Decahydrochinolinderivate, Verfahren zu deren Herstellung, Zwischenverbindungen für die Herstellung, ihre Verwendung als Arzneimittel und diese enthaltende Zusammensetzungen
New derivatives of decahydroquinoline, process for their preparation, intermediaires of the preparation, their use as medicines and compositions containing them

(30) Priorité: 24.09.1991 FR 9111736
(43) Date de publication de la demande: 31.03.1993
(73) Titulaire: ROUSSEL UCLAF, F-93230 Romainville (FR)
(72) Inventeur: Clemence, François, F-75009 Paris (FR); Hamon, Gilles, F-93340 Le Raincy (FR); Le Martret, Odile, F-75016 Paris (FR)
(74) Mandataire: Vieillefosse, Jean-Claude

(56) Documents cités:
- FR-A- 1 510 009
- FR-M- 3 657
- US-A- 2 359 329

## Description

La présente invention concerne de nouveaux dérivés de la décahydroquinoléine, leur procédé de préparation, les intermédiaires de préparation, leur application à titre de médicaments et les compositions les renfermant.

L'invention a pour objet des produits de formule (I): 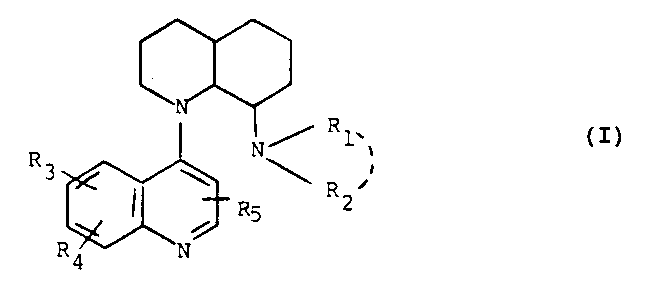
dans laquelle :
R₁ et R₂ identiques ou différents représentent un atome d'hydrogène ou un radical alkyle renfermant de 1 à 5 atomes de carbone éventuellement substitué ou bien R₁ et R₂ forment ensemble avec l'atome d'azote auquel ils sont liés un radical cyclique renfermant éventuellement un autre hétéroatome choisi parmi l'oxygène, l'azote et le soufre et éventuellement substitué par un ou plusieurs radicaux choisis parmi les radicaux alkyle et alcoxy renfermant au plus 5 atomes de carbone, les radicaux benzyle et phényle, tous ces radicaux étant eux-mêmes éventuellement substitués,
R₃ et R₄ identiques ou différents représentent un atome d'hydrogène, un atome d'halogène, un radical hydroxyle, alcoxy ou alkyle renfermant de 1 à 5 atomes de carbone et éventuellement substitués,
R₅ représente un atome d'hydrogène ou un radical alkyle renfermant de 1 à 5 atomes de carbone et éventuellement substitué,
tous les radicaux alkyle, alcoxy, benzyle et phényle tels que définis ci-dessus étant éventuellement substitués par un ou plusieurs radicaux choisis parmi les atomes d'halogène, le radical hydroxyle, les radicaux alkyle et alcoxy renfermant au plus 4 atomes de carbone et le radical phényle,
lesdits produits de formule (I) pouvant être sous toutes les formes isomères, racémiques, énantiomères et diastéréoisomères possibles et sous forme de sels d'addition avec les acides minéraux ou organiques ou de sels d'ammonium quaternaire desdits produits de formule (I).

Dans les produits de formule (I) et dans ce qui suit :
- le terme radical alkyle linéaire ou ramifié, en particulier le terme alkyle inférieur, désigne de préférence les radicaux méthyle, éthyle, n-propyle ou isopropyle, mais peut également représenter un radical n-butyle, isobutyle, sec-butyle, t-butyle ou n-pentyle,
- le terme radical alcoxy linéaire ou ramifié désigne de préférence les radicaux méthoxy ou éthoxy, mais peut aussi représenter un radical propoxy, isopropoxy, butoxy linéaire, secondaire ou tertiaire.
- le terme atome d'halogène désigne de préférence l'atome de chlore, mais peut aussi représenter un atome de fluor, de brome ou d'iode.

Dans les produits de formule (I) et dans ce qui suit, R₁ et R₂ peuvent ainsi former avec l'atome d'azote auquel ils sont liés un radical monoalkyl ou dialkylamino dans lesquels les radicaux alkyles linéaires ou ramifiés renferment de 1 à 5 atomes de carbone et peuvent ainsi représenter par exemple des radicaux méthyle, éthyle, isopropyle, n-butyle ou isobutyle ; on peut citer par exemple et de manière non exhaustive, les radicaux méthylamino, diméthylamino, éthylamino, éthylméthylamino, isopropylamino, isopropylméthylamino ou encore diisopropylamino.

Lorsque R₁ et R₂ forment ensemble avec l'atome d'azote auquel ils sont liés un hétérocycle, il s'agit, par exemple, d'un cycle pyrrolidinyle, pipéridinyle, morpholinyle ou pipérazinyle, ces radicaux étant éventuellement substitués ainsi qu'il est indiqué ci-dessus.

L'hétérocycle que peuvent former R₁ et R₂ avec l'atome d'azote auquel ils sont liés peut être substitué de préférence par un ou plusieurs substituants choisis parmi les radicaux alkyle et alcoxy linéaires ou ramifiés renfermant de 1 à 5 atomes de carbone, phényle et benzyle, tous ces radicaux étant eux-mêmes éventuellement substitués ainsi qu'il est indiqué ci-dessus.

Lorsque le radical alkyle est substitué, on peut citer par exemple les radicaux trifluorométhyle et hydroxyméthyle. On peut citer, comme exemples de tels radicaux substitués formés par R₁ et R₂ avec l'atome d'azote auquel ils sont liés, les radicaux méthylpipérazinyle, éthylpipérazinyle, propylpipérazinyle, phénylpipérazinyle, chlorophénylpipérazinyle, trifluorométhylphénylpipérazinyle ou encore benzylpipérazinyle.

Parmi les produits de formule (I), on peut citer tout particulièrement ceux dans lesquels R₃ représente un atome d'hydrogène, R₄ représente un atome de chlore en position 7 ou un radical trifluorométhyle en position 8 et R₅ représente un atome d'hydrogène et ceux dans lesquels R₃ représente un atome d'hydrogène, R₄ représente un atome de chlore en position 7 ou un radical trifluorométhyle en position 8 et R₅ représente un radical trifluorométhyle en position 1.

Les sels d'addition avec les acides minéraux ou organiques des produits de formule (I) peuvent être, par exemple, les sels formés avec les acides chlorhydrique, bromhydrique, iodhydrique, nitrique, sulfurique, phosphorique, propionique, acétique, formique, benzoïque, maléique, fumarique, succinique, tartrique, citrique, oxalique, glyoxylique, aspartique, ascorbique, les acides alkylmonosulfoniques tels que par exemple l'acide méthanesulfonique, l'acide éthanesulfonique, l'acide propanesulfonique, les acides alkyldisulfoniques tels que par exemple l'acide méthanedisulfonique, l'acide alpha, bêta-éthanedisulfonique, les acides arylmonosulfoniques tels que l'acide benzènesulfonique et les acides aryldisulfoniques.

L'invention a plus particulièrement pour objet les produits de formule (I) telle que définie ci-dessus dans laquelle R₁ et R₂ forment ensemble avec l'atome d'azote auquel ils sont liés un radical pyrrolidinyle,
R₃ et R₄ identiques ou différents représentent un atome d'hydrogène, un atome d'halogène ou un radical trifluorométhyle, R₅ représente un atome d'hydrogène ou un radical trifluorométhyle, lesdits produits de formule (I) pouvant être sous toutes les formes isomères, racémiques, énantiomères et diastéréoisomères, possibles et sous forme de sels d'addition avec les acides minéraux ou organiques ou de sels d'ammonium quaternaire desdits produits de formule (I).

L'invention a tout particulièrement pour objet les produits de formule I telle que définie ci-dessus dont les noms suivent :
- la (4a alpha, 8 alpha, 8a alpha) (±) 1-(7-chloro 4-quinoléinyl) décahydro 8-(1-pyrrolidinyl) quinoléine
- le dichlorhydrate de (4a alpha, 8 alpha, 8a alpha) (±) 1-(7-chloro 4-quinoléinyl) décahydro 8-(1-pyrrolidinyl) quinoléine
ainsi que leurs sels d'addition avec les acides minéraux ou organiques ou leurs sels d'ammonium quaternaire.

L'invention a également pour objet un procédé de préparation des produits de formule (I) telle que définie ci-dessus caractérisé en ce que l'on fait réagir un composé de formule (II): 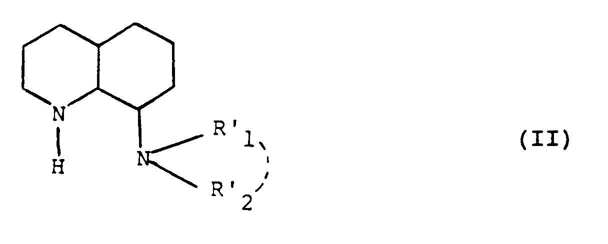
dans laquelle R′₁ et R′₂ ont la signification indiquée ci-dessus pour R₁ et R₂ dans lesquelles les éventuelles fonctions réactives sont éventuellement protégées, soit l'un ou les deux de R′₁ et R′₂ représente(nt) un groupement protecteur du radical amino ou monoalkylamino,
avec un composé de formule (III): 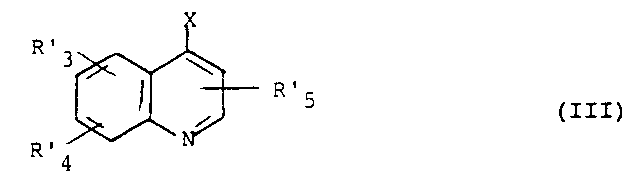
dans laquelle R′₃, R′₄ et R′₅ ont les significations ci-dessus respectivement pour R₃, R₄ et R₅ dans lesquelles les éventuelles fonctions réactives sont éventuellement protégées et X représente un atome d'halogène, ou un groupe partant, pour obtenir des produits de formule (IV): 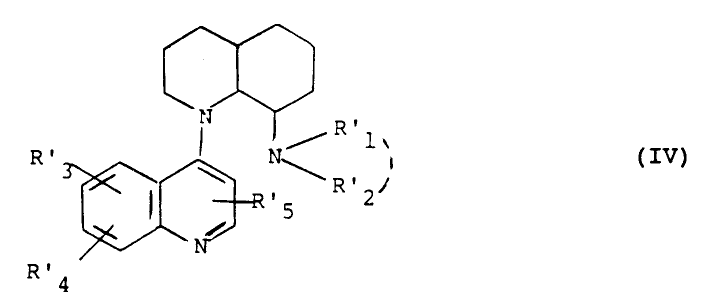
dans laquelle R′₁, R′₂, R′₃, R′₄ et R′₅ ont les significations indiquées ci-dessus,
produits de formule (IV) que l'on peut soumettre si nécessaire et si désiré à l'une ou plusieurs des réactions suivantes, dans un ordre quelconque:
- une réaction d'élimination des groupements protecteurs que peuvent porter les fonctions réactives protégées,
- une réaction de salification par un acide minéral ou organique pour obtenir le sel correspondant,
- une réaction de dédoublement des formes racémiques en produits dédoublés,
lesdits produits de formule (I) ainsi obtenus étant sous toutes les formes isomères, racémiques, énantiomères et diastéréoisomères, possibles et sous forme de sels d'addition avec les acides minéraux ou organiques ou de sels d'ammonium quaternaire desdits produits de formule (I).

Dans des conditions préférentielles de mise en oeuvre de l'invention, le procédé ci-dessus peut être réalisé par chauffage au reflux des produits de formules (II) et (III) dans un solvant tel que par exemple le diméthylformamide ou encore le diméthylformamide avec du carbonate de sodium ou encore de l'éthoxyéthanol en présence de bicarbonate de sodium, la réaction étant réalisée de préférence en présence de sulfate de cuivre.

Dans les produits de formule (III), X peut représenter un atome d'halogène tel qu'un atome de chlore ou de brome et de préférence un atome de chlore. X peut également représenter un groupe partant tel que par exemple un radical mésyle ou tosyle.

Les produits de formule (I) peuvent être représentés par les produits de formule (IV) ou être obtenus par élimination des groupements protecteurs des fonctions réactives que peuvent porter ces produits de formule (IV).

Les diverses fonctions réactives qui peuvent, si nécessaire, être protégées de manière appropriée, sont par exemple, les fonctions hydroxyle que peuvent porter les radicaux R₁ , R₂ , R₃ , R₄ et R₅ ou représenter R₃ et R₄ ; amino et monoalkylamino que peuvent représenter -N(R1)(R2).

La liste suivante, non exhaustive, d'exemples de protection de fonctions réactives peut être citée :
- les groupements hydroxyle peuvent être protégés par exemple par les radicaux triméthylsilyle, dihydropyranne, benzyle, méthoxy ou méthoxyméthyle,
- les groupements amino peuvent être protégés par exemple par les radicaux trityle, benzyle, ter butoxycarbonyle, phtalimido ou d'autres radicaux connus dans la chimie des peptides,
L'élimination de ces groupements protecteurs est effectuée dans les conditions usuelles connues de l'homme de métier notamment l'hydrolyse acide, effectuée avec un acide tel que l'acide chlorhydrique, benzène sulfonique ou para-toluène sulfonique, formique ou trifluoroacétique.
Le groupement phtalimido est éliminé par l'hydrazine.
On trouvera une liste de différents groupements protecteurs utilisables par exemple dans le brevet BF 2 499 995.

Les produits décrits ci-dessus peuvent, si désiré, faire l'objet de réaction de salification pour donner des sels d'acide minéral ou organique ou encore des sels d'ammonium quaternaire par exemple par les méthodes usuelles connues de l'homme de métier.

Les formes optiquement actives des produits de formule (I) peuvent être préparées par dédoublement des racémiques selon les méthodes usuelles.

Les produits de formule (I) tels que définis ci-dessus ainsi que leurs sels d'addition avec les acides ou les sels d'ammonium quaternaire, présentent d'intéressantes propriétés pharmacologiques. Ils présentent en particulier une forte affinité pour les récepteurs opiacés et notamment pour les récepteurs kappa et sont doués de propriétés analgésiques centrales.

Ils sont doués également de propriétés diurétiques et certains d'entre eux possèdent des propriétés anti-arythmiques, anti-ischémiques et hypotensives.

Les produits de formule (I) possèdent des propriétés anti-parasitaires et notamment anti-paludique.

Ces propriétés justifient leur application en thérapeutique et l'invention a également pour objet à titre de médicaments, les produits de formule (I) telle que définie ci-dessus, lesdits produits de formule (I) étant sous toutes les formes isomères, racémiques, énantiomères et diastéréoisomères possibles et sous forme de sels d'addition avec les acides minéraux ou organiques ou de sels d'ammonium quaternaire pharmaceutiquement acceptables desdits produits de formule (I).

L'invention a plus particulièrement pour objet, à titre de médicament, les produits de formule (I) telle que définie ci-dessus dans laquelle R₁ et R₂ forment ensemble avec l'atome d'azote auquel ils sont liés un radical pyrrolidinyle, R₃, R₄ et R₅ identiques ou différents représentent un atome d'hydrogène, un atome d'halogène ou un radical trifluorométhyle, lesdits produits de formule (I) pouvant être sous toutes les formes isomères, racémiques, énantiomères et diastéréoisomères, possibles et sous forme de sels d'addition avec les acides minéraux ou organiques ou de sels d'ammonium quaternaire pharmaceutiquement acceptables desdits produits de formule (I).

L'invention a plus particulièrement pour objet, à titre de médicament, les produits de formule (I) suivants:
- le (4a alpha, 8 alpha, 8a alpha) (±) 1-(7-chloro 4-quinoléinyl) décahydro 8-(1-pyrrolidinyl) quinoléine
- le dichlorhydrate de (4a alpha, 8 alpha, 8a alpha) (±) 1-(7-chloro 4-quinoléinyl) décahydro 8-(1-pyrrolidinyl) quinoléine
ainsi que leurs sels d'addition avec les acides minéraux ou organiques pharmaceutiquement acceptables ou leurs sels d'ammonium quaternaire.

Les médicaments, objet de l'invention, permettent notamment de soulager une douleur quelle qu'en soit l'origine, par exemple une douleur de nature musculaire, articulaire ou nerveuse.

Ils peuvent être aussi utilisés dans le traitement des douleurs dentaires, des migraines, du zona, dans le traitement des douleurs intenses, en particulier rebelles aux antalgiques périphériques, par exemple au cours du processus néoplasique, dans le traitement des pancréatites, coliques néphrétiques ou biliaires, dans le traitement des douleurs post-opératoires et post-traumatiques.

Ils peuvent également être utilisés dans le traitement des insuffisances cérébrales d'origine ischémique dans les troubles de la mémoire et de l'attention.

Les médicaments objet de l'invention peuvent notamment être utilisés dans le traitement du paludisme.

La posologie varie notamment en fonction de la voie d'administration, de l'affection traitée et du sujet en cause. Par exemple, chez l'adulte, elle peut varier entre 20 et 1000 mg de principe actif par jour, par voie orale et entre 5 et 100 mg de principe actif par jour, par voie parentérale.

Les médicaments, objet de l'invention, trouvent aussi leur emploi dans le traitement des arythmies.
La dose usuelle, variable selon le dérivé utilisé, le sujet traité et l'affection en cause, peut être, par exemple, de 50 mg à 1 g par jour.

Par voie orale, le principe actif peut être administré à la dose quotidienne de 200 à 800 mg, par exemple, dans le traitement des arythmies ventriculaires, supraventriculaires et jonctionnelles, soit environ de 3 et 12 mg par kilogramme de poids corporel.

Les médicaments, objet de l'invention, peuvent aussi être utilisés dans le traitement des syndromes oedémateux, de l'insuffisance cardiaque, de certaines obésités, des cyrrhoses, dans le traitement des oedèmes sévères et réfractaires, en particulier ceux de l'insuffisance cardiaque congestive et dans le traitement au long cours de l'hypertension artérielle.

La dose quotidienne de principe actif est variable. Elle peut être par exemple de 60 à 100 mg par jour par voie orale.

L'invention s'étend aux compositions pharmaceutiques renfermant comme principe actif les médicaments définis ci-dessus.

Ces compositions pharmaceutiques peuvent être administrées par voie buccale, rectale, par voie parentérale ou par voie locale en application topique sur la peau et les muqueuses.

Ces compositions peuvent être solides ou liquides et se présenter sous toutes les formes pharmaceutiques couramment utilisées en médecine humaine comme, par exemple, les comprimés simples ou dragéifiés, les gélules, les granulés, les suppositoires, les préparations injectables, les pommades, les crèmes, les gels et les préparations en aérosols ; elles sont préparées selon les méthodes usuelles. Le principe actif peut y être incorporé à des excipients habituellement employés dans ces compositions pharmaceutiques, tels que le talc, la gomme arabique, le lactose, l'amidon, le stéarate de magnésium, le beurre de cacao, les véhicules aqueux ou non, les corps gras d'origine animale ou végétale, les dérivés paraffiniques, les glycols, les divers agents mouillants, dispersants ou émulsifiants, les conservateurs.

Les produits de départ de formule (II) peuvent être obtenus, par exemple, ainsi qu'il est indiqué dans le brevet français n° 2592879.

Certains produits de départ de formule (III) sont commerciaux tel que par exemple la 4,7-dichloroquinoléine.

Les autres produits de départ de formule (III) peuvent être préparés par des méthodes comparables ou connues de l'homme de métier. On peut citer par exemple le brevet allemand DE 28 06 909 (Méfloquine) ou le brevet français BF 1 584 746 (Floctafénine).

Les exemples donnés ci-aprés illustrent l'invention sans toutefois la limiter.

### Exemple 1: (4a alpha, 8 alpha, 8a alpha) (±) 1-(7-chloro 4-quinoléinyl) décahydro 8-(1-pyrrolidinyl) quinoléine

On introduit à 20°C, 7,13 g de 4,7-dichloroquinoléine et 6,25 g de [4a(RS) (4a alpha, 8 alpha, 8a alpha) (±)] décahydro 8-(1- pyrrolidinyl) quinoléine (obtenu selon BF 2.592.879) dans 30 ml de diméthylformamide en présence de 206 mg de sulfate de cuivre anhydre. On porte la solution au reflux sous agitation et maintient durant environ 2 heures. On distille sous pression réduite , refroidit à la température ambiante et dilue par 100 ml d'eau puis alcalinise à environ pH 10 avec du carbonate de sodium. On essore, rince par 3 x 50 ml d'eau et reprend l'insoluble par 100 ml de chlorure de méthylène. On lave par 100 ml d'eau, sèche, filtre, rince et distille sous pression réduite. On reprend l'extrait sec dans 80 ml d'éther éthylique à la température ambiante, filtre et sèche sous pression réduite. L'extrait sec est empâté dans 10 ml d'éther isopropylique: on essore, rince à l'éther isopropylique, sèche sous vide à environ 50°C et obtient 1,66 gr de produit attendu. ( F=136°C -138°C) sous forme de base.

### Exemple 2: dichlorhydrate de (4a alpha, 8 alpha, 8a alpha) (+-) 1-(7-chloro 4-quinoléinyl) décahydro 8-(1-pyrrolidinyl) quinoléine

On opère à chaud et dissout 1,66 gr du produit obtenu à l'exemple 1 dans 8 ml d'isopropanol, filtre et rince avec 7 ml d'isopropanol. On ajoute 2 ml de solution de chlorure d'hydrogène dans l'éthanol (environ 6,6N). On amorce la cristallisation, essore à la tempétature ambiante, rince par 3 x 2 ml d'isopropanol et 3 x 5 ml d'éther éthylique, recristallise dans 30 ml d'isopropanol à 1,5 % d'eau, puis à nouveau dans 7 ml d'éthanol 100. On sèche à la température ambiante et obtient 1,3 gr de produit attendu.(F = 220°C) RMN dans CDCl3
3,78 (m) C6H5-N-CH 1H
8,53 (d) H en 2 sur quinoléine
6,75 (d) H en 3 sur quinoléine
7,32 (dd) H en 6 sur quinoléine
7,88 (dd) H en 5 sur quinoléine
de 0,8 à 3,4 les autres protons

### Exemple 3:

On a préparé des comprimés répondant à la formule suivante:

| | |
|---|---|
| - produit de l'exemple 2 | 200 mg |
| _ excipient q.s.p. | 800 mg |
| (détail de l'excipient: lactose, talc, amidon, stéarate de magnésium) | |

### Exemple 4:

On a préparé un soluté injectable (voie intra-musculaire) répondant à la formule suivante:

| | |
|---|---|
| - produit de l'exemple 2 | 50 mg |
| - solvant stérile q.s.p. | 10 ml |

### Etude pharmacologique

### 1) Liaison au récepteur opiacé kappa in vitro

On utilise des culots membranaires conservés à - 30°C pendant environ 30 jours et préparés à partir de cervelets de cobayes.

Ces culots sont remis en suspension dans le tampon Tris pH 7,7. On répartit des fractions de 2 ml dans des tubes à hémolyse et ajoute de la 9³H Ethylkétocyclazocine 1nM et le produit à étudier (le produit est d'abord testé à 5X10⁻⁶M (en triple). Lorsque le produit testé déplace de plus de 50 % la radioactivité liée spécifiquement au récepteur, il est testé à nouveau selon une gamme de 7 concentrations afin de déterminer la concentration qui inhibe de 50 % la radioactivité liée spécifiquement au récepteur. On détermine ainsi la concentration inhibitrice 50 %).

La liaison non spécifique est déterminée par addition de produit connu sous le nom U-50488 H à 10⁻⁵M (en triple). On incube à 25°C pendant 40 minutes, remet au bain-marie à 0°C, 5 minutes, filtre sous pression réduite, rince au tampon Tris pH 7,7 et compte la radioactivité en présence de scintillant Triton ^{(R)}.

Les résultats sont exprimés directement en concentration inhibitrice 50 % (CI₅₀), (c'est-à-dire en concentration de produit étudié, exprimée en nM, nécessaire pour déplacer 50 % de la radioactivité spécifique fixée sur le récepteur étudié.

### Résultats :

| Produit de l'exemple | CI₅₀ en nM |
|---|---|
| 2 | 30600 |

### 2) Test de détermination in vitro de l'activité anti-paludique

La culture "in vitro" de Plasmodium falciparum est effectuée en cupules, sur plaques de polystyrène utilisées pour culture de tissus. Elle est réalisée à 37°C dans une étuve à CO₂ selon les techniques de TRAGER et JENSEN (1976 - 1978) sur un milieu RPMI 1640 modifié (AZOULAY et al 1983) tamponné avec un mélange de tampon HEPES (40 m.moles/l), de glucose (4 g/l) et 10 pour cent de sérum humain.

L'activité antimalarique "in vitro" des différents produits testés a été étudiée sur deux souches différentes de Plasmodium falciparum
- une souche FCC₂ sensible à la chloroquine
- une souche FZ résistance à la chloroquine (résistance de type R₃).

### Mesure de l'inhibition de la prolifération en culture continue (Trager - Polonsky 1981)

Ce test permet de mesurer l'action du produit à tester sur la maturation du trophozoïte en schizonte, mais également la possibilité de repénétration des mérozoïtes, libérés par éclatement des corps en rosace, dans les globules rouges sains, et leur transformation en trophozoïtes.

Les souches de Plasmodium falciparum utilisées, effectuent un cycle complet en 48 heures.

Le milieu RPS, renfermant 40 mM de tampon H.E.P.E.S. et enrichi par 2 g de glucose permet un bon développement de la culture, sans renouvellement de milieu durant deux jours.

Les hématies parasitées sont mises en suspension dans le milieu RPS 10 contenant le produit à tester à la concentration choisie.

La parasitémie de départ se situe entre 0,2 et 0,3 %.
. 0,7 ml de la suspension globulaire sont déposés dans chaque cupule.
. chaque concentration du produit choisi est testée sur 3 cupules.
. Dans chaque multiplaque utilisée, trois cupules témoins renfermant la suspension globulaire dans le milieu RPS 10 sans produit, permettent d'obtenir le taux maximum de prolifération de la culture en 48 heures.
. Après un temps d'incubation de 48 heures, sans renouvellement du milieu, en étuve humide avec un taux de CO₂ de 5 %, le taux de parasitémie pour chaque cupule est évalué.

Pour cela, les boîtes, retirées de l'étuve, sont laissées 10 minutes au repos sous la hotte à flux laminaire.

Le liquide surnageant est prélevé et un frottis est effectué après avoir homogénéisé le tapis d'hématies restant au fond de la cupule. Sur ces frottis, fixés et colorés, les différentes formes du parasite sont comptées pour 2000 à 3000 hématies par lame.

Les résultats sont exprimés en pourcentage de prolifération par rapport aux témoins.

La relation graphique effectuée entre le pourcentage de parasitémie par rapport aux témoins et les différentes concentrations de produits utilisés, permet de calculer la DE 50 (Dose Efficace 50 %).

### Résultats :

| Produit de l'exemple | Souche FCC₂ sensible à la chloroquine DE₅₀ (µg/ml) | Souche FZ résistante à la chloroquine DE₅₀ (µg/ml) |
|---|---|---|
| 2 | 0,35 | 0,34 |

## Revendications

1. Produits de formule (I): 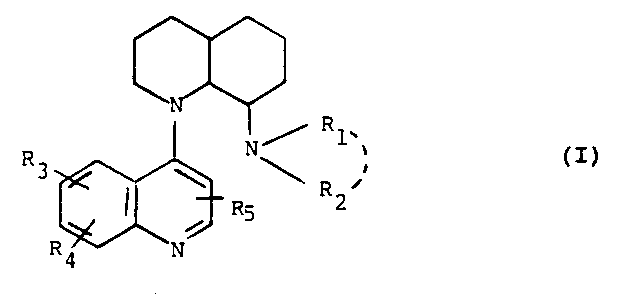 dans laquelle
R₁ et R₂ identiques ou différents représentent un atome d'hydrogène ou un radical alkyle renfermant de 1 à 5 atomes de carbone éventuellement substitué ou bien R₁ et R₂ forment ensemble avec l'atome d'azote auquel ils sont liés un radical cyclique renfermant éventuellement un autre hétéroatome choisi parmi l'oxygène, l'azote et le soufre et éventuellement substitué par un ou plusieurs radicaux choisis parmi les radicaux alkyle et alcoxy renfermant au plus 5 atomes de carbone, les radicaux benzyle et phényle, tous ces radicaux étant eux-mêmes éventuellement substitués,
R₃ et R₄ identiques ou différents représentent un atome d'hydrogène, un atome d'halogène, un radical hydroxyle, alcoxy ou alkyle renfermant de 1 à 5 atomes de carbone et éventuellement substitués,
R₅ représente un atome d'hydrogène ou un radical alkyle renfermant de 1 à 5 atomes de carbone et éventuellement substitué,
tous les radicaux alkyle, alcoxy, benzyle et phényle tels que définis ci-dessus étant éventuellement substitués par un ou plusieurs radicaux choisis parmi les atomes d'halogène, le radical hydroxyle, les radicaux alkyle et alcoxy renfermant au plus 4 atomes de carbone et le radical phényle,
lesdits produits de formule (I) pouvant être sous toutes les formes isomères, racémiques, énantiomères et diastéréoisomères, possibles et sous forme de sels d'addition avec les acides minéraux ou organiques ou de sels d'ammonium quaternaire desdits produits de formule (I).

2. Produits de formule (I) telle que définie à la revendication 1 dans laquelle R₁ et R₂ forment ensemble avec l'atome d'azote auquel ils sont liés un radical pyrrolidinyle, R₃ et R₄ identiques ou différents représentent un atome d'hydrogène, un atome d'halogène ou un radical trifluorométhyle, R₅ représente un atome d'hydrogène ou un radical trifluorométhyle, lesdits produits de formule (I) pouvant être sous toutes les formes isomères, racémiques, énantiomères et diastéréoisomères, possibles et sous forme de sels d'addition avec les acides minéraux ou organiques ou de sels d'ammonium quaternaire desdits produits de formule (I).

3. Les produits de formule (I) telle que définie à la revendication 1 suivants :
- La (4a alpha, 8 alpha, 8a alpha) (±) 1-(7-chloro 4-quinoléinyl) décahydro 8-(1-pyrrolidinyl) quinoléine
- le dichlorhydrate de (4a alpha, 8 alpha, 8a alpha) (±) 1-(7-chloro 4-quinoléinyl) décahydro 8-(1-pyrrolidinyl) quinoléine
ainsi que leurs sels d'addition avec les acides minéraux ou organiques ou leurs sels d'ammonium quaternaire.

4. Procédé de préparation des produits de formule (I) telle que définie à la revendication 1 caractérisé en ce que l'on fait réagir un composé de formule (II): 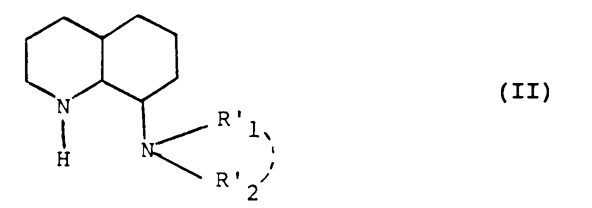 dans laquelle R′₁ et R′₂ ont la signification indiquée à la revendication 1 pour R₁ et R₂ dans lesquelles les éventuelles fonctions réactives sont éventuellement protégées, soit l'un ou les deux de R′₁ et R′₂ représente(nt) un groupement protecteur du radical amino ou monoalkylamino,
avec un composé de formule (III): 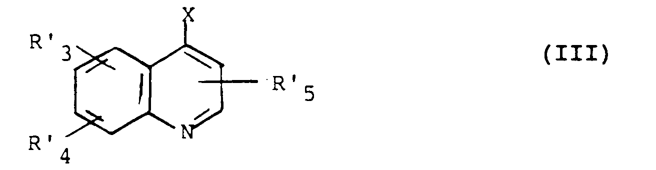 dans laquelle R′₃, R′₄ et R′₅ ont les significations indiquées à la revendication 1 respectivement pour R₃, R₄ et R₅ dans lesquelles les éventuelles fonctions réactives sont éventuellement protégées et X représente un atome d'halogène ou un groupe partant, pour obtenir des produits de formule (IV): 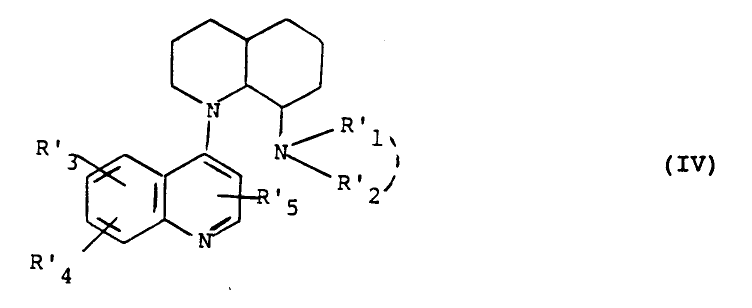 dans laquelle R′₁, R′₂, R′₃, R′₄ et R′₅ ont les significations indiquées ci-dessus,
produits de formule (IV) que l'on peut soumettre si nécessaire et si désiré à l'une ou plusieurs des réactions suivantes, dans un ordre quelconque:
- une réaction d'élimination des groupements protecteurs que peuvent porter les fonctions réactives protégées,
- une réaction de salification par un acide minéral ou organique pour obtenir le sel correspondant,
- une réaction de dédoublement des formes racémiques en produits dédoublés,
lesdits produits de formule (I) ainsi obtenus étant sous toutes les formes isomères, racémiques, énantiomères et diastéréoisomères, possibles et sous forme de sels d'addition avec les acides minéraux ou organiques ou de sels d'ammonium quaternaire desdits produits de formule (I).

5. A titre de médicaments, les produits de formule (I) telle que définie à la revendication 1,
lesdits produits de formule (I) ainsi obtenus étant sous toutes les formes isomères, racémiques, énantiomères et diastéréoisomères, possibles et sous forme de sels d'addition avec les acides minéraux ou organiques pharmaceutiquement acceptables ou de sels d'ammonium quaternaire desdits produits de formule (I).

6. A titre de médicaments , les produits de formule (I) telle que définie aux revendications 2 et 3,
lesdits produits de formule (I) ainsi obtenus étant sous toutes les formes isomères, racémiques, énantiomères et diastéréoisomères, possibles et sous forme de sels d'addition avec les acides minéraux ou organiques pharmaceutiquement acceptables ou de sels d'ammonium quaternaire desdits produits de formule (I).

7. Les compositions pharmaceutiques contenant à titre de principe actif l'un au moins des médicaments tels que définis à l'une quelconque des revendications 5 ou 6.

## Claims

1. The products of formula (I): 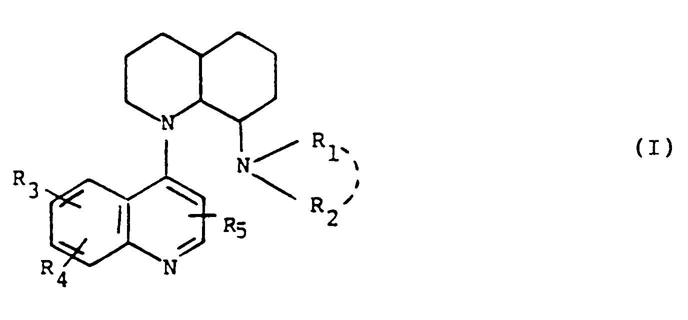 in which:
R₁ and R₂, identical or different, represent a hydrogen atom or an optionally substituted alkyl radical containing 1 to 5 carbon atoms or R₁ and R₂ form together with the nitrogen atom to which they are linked a cyclic radical optionally containing another heteroatom chosen from oxygen, nitrogen and sulphur and optionally substituted by one or more radicals chosen from alkyl and alkoxy radicals containing at most 5 carbon atoms, benzyl and phenyl radicals, all these radicals being themselves optionally substituted,
R₃ and R₄, identical or different, represent a hydrogen atom, a halogen atom, a hydroxyl, alkoxy or alkyl radical containing 1 to 5 carbon atoms and optionally substituted,
R₅ represents a hydrogen atom or an alkyl radical containing 1 to 5 carbon atoms and optionally substituted,
all the alkyl, alkoxy, benzyl and phenyl radicals as defined above being optionally substituted by one or more radicals chosen from halogen atoms, the hydroxyl radical, alkyl and alkoxy radicals containing at most 4 carbon atoms and the phenyl radical,
the said products of formula (I) being able to be in all possible racemic, enantiomeric and diastereoisomeric isomer forms and in the form of addition salts with mineral or organic acids or quaternary ammonium salts of said products of formula (I).

2. The products of formula (I) as defined in claim 1 in which R₁ and R₂ form together with the nitrogen atom to which they are linked a pyrrolidinyl radical, R₃ and R₄ identical or different represent a hydrogen atom, a halogen atom or a trifluoromethyl radical, R₅ represents a hydrogen atom or a trifluoromethyl radical, the said products of formula (I) being able to be in all possible racemic, enantiomeric and diastereoisomeric isomer forms and in the form of addition salts with mineral or organic acids or quaternary ammonium salts of said products of formula (I).

3. The following products of formula (I) as defined in claim 1:
(4a alpha, 8 alpha, 8a alpha) (±) 1-(7-chloro-4-quinolinyl)-decahydro-8-(1-pyrrolidinyl) quinoline
- (4a alpha, 8 alpha, 8a alpha) (±) 1-(7-chloro-4-quinolinyl)-decahydro-8-(1-pyrrolidinyl) quinoline dihydrochloride.
as well as their addition salts with mineral or organic acids or their quaternary ammonium salts.

4. Preparation process for the products of formula (I) as defined in claim 1 characterized in that a compound of formula (II): 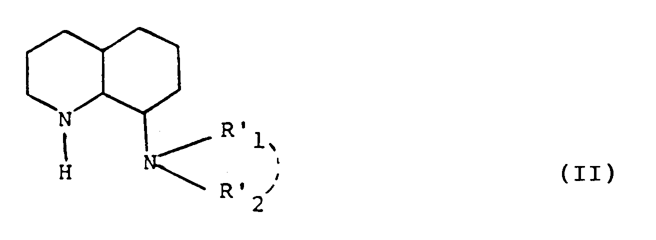 in which R'₁ and R'₂ have the meaning indicated in claim 1 for R₁ and R₂ in which the optional reactive functions are optionally protected, or one or both of R'₁ and R'₂ represent(s) a protector group of the amino or monoalkylamino radical,
is reacted with a compound of formula (III): 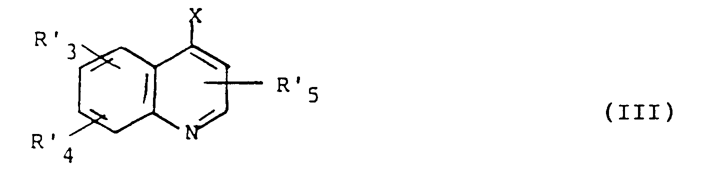 in which R'₃, R'₄ and R'₅ have the meanings indicated in claim 1 for R₃, R₄ and R₅ respectively in which the optional reactive functions are optionally protected and X represents a halogen atom, or a parting group, in order to obtain the products of formula (IV): 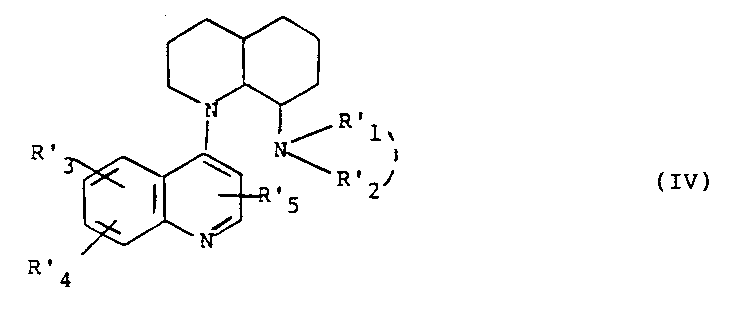 in which R'₁, R'₂, R'₃, R'₄ and R'₅ have the meanings indicated above,
which products of formula (IV) can be subjected, if necessary and if desired, to one or several of the following reactions, in any order:
- an elimination reaction of the protective groups that can be carried by the protected reactive functions,
- a salification reaction by a mineral or organic acid in order to obtain the corresponding salt,
- a resolution reaction of racemic forms into resolved products,
the said products of formula (I) thus obtained being in all possible racemic, enantiomeric and diastereoisomeric isomer forms and in the form of addition salts with mineral or organic acids or quaternary ammonium salts of said products of formula (I).

5. As medicaments, the products of formula (I) as defined in claim 1,
the said products of formula (I) thus obtained being in all possible racemic, enantiomeric and diastereoisomeric isomer forms and in the form of addition salts with pharmaceutically acceptable mineral or organic acids or quaternary ammonium salts of said products of formula (I).

6. As medicaments, the products of formula (I) as defined in claims 2 and 3,
the said products of formula (I) thus obtained being in all possible racemic, enantiomeric and diastereoisomeric isomer forms and in the form of addition salts with pharmaceutically acceptable mineral or organic acids or quaternary ammonium salts of said products of formula (I).

7. The pharmaceutical compositions containing as active ingredient at least one of the medicaments defined in any one of claims 5 or 6.

## Patentansprüche

1. Produkte der Formel (I): 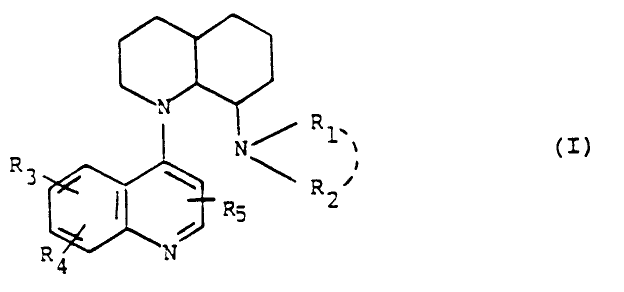 worin
R₁ und R₂, die identisch oder verschieden sind, ein Wasserstoffatom oder einen Alkylrest mit 1 bis 5 Kohlenstoffatomen, gegebenenfalls substituiert, bedeuten oder auch bilden R₁ und R₂ zusammen mit dem Stickstoffatom, an das sie gebunden sind, einen cyclischen Rest, der gegebenenfalls ein weiteres Heteroatom, ausgewählt unter Sauerstoff, Stickstoff und Schwefel, enthält, und gegebenenfalls substituiert ist durch einen oder mehrere Rest(e), ausgewählt unter den Alkyl- und Alkoxyresten mit höchstens 5 Kohlenstoffatomen, den Benzyl- und Phenylresten, wobei alle diese Reste selbst gegebenenfalls substituiert sind,
R₃ und R₄, die identisch oder verschieden sind, bedeuten ein Wasserstoffatom, ein Halogenatom, einen Hydroxyl-, Alkoxy- oder Alkylrest mit 1 bis 5 Kohlenstoffatomen, und gegebenenfalls substituiert,
R₅ bedeutet ein Wasserstoffatom oder einen Alkylrest mit 1 bis 5 Kohlenstoffatomen, und gegebenenfalls substituiert, wobei alle die Alkyl-, Alkoxy-, Benzyl- und Phenylreste, wie sie vorstehend definiert sind, gegebenenfalls substituiert sind durch einen oder mehrere Rest(e), ausgewählt unter den Halogenatomen, dem Hydroxylrest, den Alkyl- und Alkoxyresten mit höchstens 4 Kohlenstoffatomen und dem Phenylrest,
wobei diese Produkte der Formel (I) unter allen möglichen isomeren, racemischen, enantiomeren und diastereoisomeren Formen vorliegen können, und in Form von Salzen mit Mineral- oder organischen Säuren oder quaternären Ammoniumsalzen dieser Produkte der Formel (I).

2. Produkte der Formel (I), wie sie in Anspruch 1 definiert sind, worin R₁ und R₂ zusammen mit dem Stickstoffatom, an das sie gebunden sind, einen Pyrrolidinylrest bilden, R₃ und R₄, die identisch oder verschieden sind, bedeuten ein Wasserstoffatom, ein Halogenatom, oder einen Trifluormethylrest, R₅ bedeutet ein Wasserstoffatom oder einen Trifluormethylrest, wobei diese Produkte der Formel (I) unter allen möglichen isomeren, racemischen, enantiomeren und diastereoisomeren Formen sowie in Form von Additionssalzen mit Mineral- oder organischen Säuren oder von quaternären Ammoniumsalzen dieser Produkte der Formel (I) vorliegen können.

3. Die folgenden Produkte der Formel (I) wie in Anspruch 1 definiert :
- das (4a alpha, 8 alpha, 8a alpha)(±)-1-(7-Chloro-4-chinolinyl)-decahydro-8-(1-pyrrolidinyl)-chinolin
- das (4a alpha, 8 alpha, 8a alpha)(±)-1-(7-Chloro-4-chinolinyl)-decahydro-8-(1-pyrrolidinyl)-chinolin-dihydrochlorid
sowie deren Additionssalze mit Mineral- oder organischen Säuren oder ihre quaternären Ammoniumsalze.

4. Verfahren zur Herstellung der Produkte der Formel (I), wie in Anspruch 1 definiert, dadurch gekennzeichnet, daß man eine Verbindung der Formel (II): 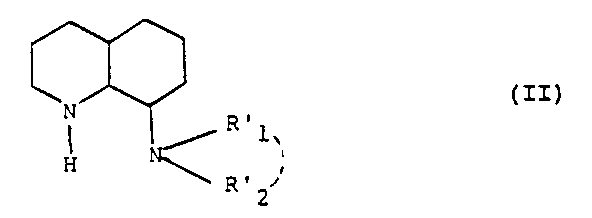 worin R'₁ und R'₂ die in Anspruch 1 für R₁ und R₂ angegebene Bedeutung haben, worin die eventuellen reaktiven Funktionen gegebenenfalls geschützt sind, wobei entweder eines oder beide R'₁ und R'₂ bedeutet/bedeuten eine Schutzgruppe des Amino- oder Monoalkylaminorestes, mit einer Verbindung der Formel (III): 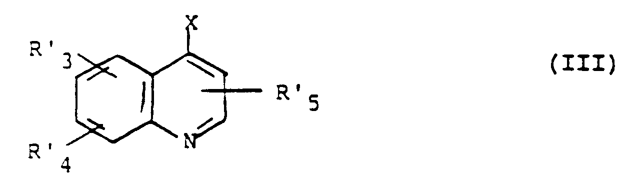 zur Reaktion bringt, worin R'₃, R'₄ und R'₅ die in Anspruch 1 bezüglich R₃, R₄ und R₅ angegebenen Bedeutungen haben, worin die eventuellen reaktionsfähigen Funktionen gegebenenfalls geschützt sind, und X ein Halogenatom oder eine austretende Gruppe bedeutet, um Produkte der Formel (IV): 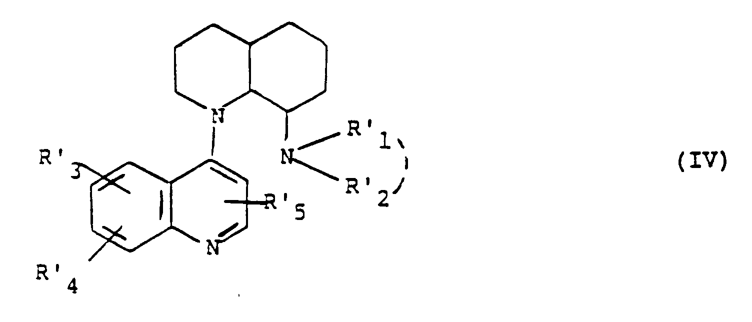 zu erhalten, worin R'₁, R'₂, R'₃, R'₄ und R'₅ die oben angegebenen Bedeutungen haben,
wobei man die Produkte der Formel (IV), falls notwendig und erwünscht, einer oder mehreren der folgenden Reaktionen in beliebiger Reihenfolge unterwerfen kann:
- eine Entfernungsreaktion der Schutzgruppen, welche die geschützten reaktionsfähigen Funktionen tragen können,
- eine Salzbildungsreaktion mit einer Mineral- oder organischen Säure, um das entsprechende Salz zu erhalten,
- eine Spaltungsreaktion der racemischen Formen in gespaltene Produkte,
wobei die so erhaltenen Produkte der Formel (I) in allen möglichen isomeren, racemischen, enantiomeren und diastereoisomeren Formen und in Form von Additionssalzen mit Mineral- oder organischen Säuren oder quaternären Ammoniumsalzen dieser Produkte der Formel (I) vorliegen.

5. Als Arzneimittel die Produkte der Formel (I), wie in Anspruch 1 definiert,
wobei die so erhaltenen Produkte der Formel (I) in allen möglichen isomeren, racemischen, enantiomeren und diastereoisomeren Formen und in Form von Additionssalzen mit pharmazeutisch annehmbaren Mineral- oder organischen Säuren oder als quaternäre Ammoniumsalze dieser Produkte der Formel (I) vorliegen.

6. Als Arzneimittel die Produkte der Formel (I), wie in den Ansprüchen 2 und 3 definiert,
wobei die so erhaltenen Produkte der Formel (I) in allen möglichen isomeren, racemischen, enantiomeren und diastereoisomeren Formen und in Form von Additionssalzen mit pharmazeutisch annehmbaren Mineral- oder organischen Säuren oder als quaternäre Ammoniumsalze dieser Produkte der Formel (I) vorliegen.

7. Pharmazeutische Zusammensetzungen, enthaltend als Wirkstoff wenigstens eines der Arzneimittel, wie sie in einem der Ansprüche 5 oder 6 definiert sind.
